Europäisches Patentamt

European Patent Office

Office européen des brevets

(11) Publication number: **0 011 439**
**B1**

## EUROPEAN PATENT SPECIFICATION

(45) Date of publication of patent specification: 05.05.82

(21) Application number: 79302496.9

(22) Date of filing: 07.11.79

(51) Int. Cl.³: **C 07 B 1/00, C 07 C 45/62, C 07 C 49/203, C 07 C 29/17, C 07 C 33/025**

(54) Method for selective preparation of cis isomers of ethylenically unsaturated compounds.

(30) Priority: 08.11.78 JP 137543/78

(43) Date of publication of application:
28.05.80 Bulletin 80/11

(45) Publication of the grant of the patent:
05.05.82 Bulletin 82/18

(84) Designated Contracting States:
CH DE FR GB NL

(56) References cited:
CH - A - 382 719
DE - C - 823 292

CHEMICAL ABSTRACTS
Vol. 79, 1973 page 314
abstract No. 115042p
Columbus, Ohio, USA
V.A. NAIDIN et al.: "Hydrogenation of
dimethylethynylcarbinol on a palladium-
aluminum oxide catalyst modified with
specifically adsorbing zinc and cadmium
cations"

(73) Proprietor: Shin-Etsu Chemical Co., Ltd.
6-1, Otemachi 2-chome
Chiyoda-ku, Tokyo (JP)

(72) Inventor: Ohno, Shigeru
4-6-15, Kamakura-shi-Dai
Kanagawa-ken (JP)
Inventor: Tezuka, Haruya
804-85, Ohya Omiya-shi
Saitama-ken (JP)
Inventor: Ishihara, Toshinobu
2-1-3 Minato-cho Jotsu-shi
Niigata-Ken (JP)

(74) Representative: Perry, Robert Edward et al,
GILL JENNINGS & EVERY 53-64 Chancery Lane
London WC2A 1HN (GB)

(56) References cited:
CHEMICAL ABSTRACTS
Vol. 83, 1975, page 430,
abstract No. 85624t
Columbus, Ohio, USA

Courier Press, Leamington Spa, England.

# Method for selective preparation of cis isomers of ethylenically unsaturated compounds

The present invention relates to a method for the selective preparation of *cis* isomers of ethylenically unsaturated compounds by the partial hydrogenation of the corresponding acetylenically unsaturated compounds, using a partially inactivated catalyst.

Various *cis* isomers of ethylenically unsaturates compounds are important in the perfumery, agricultural chemical, medical and similar fields, and an important method for their preparation is the partial hydrogenation of the triple bond in acetylenically unsaturated compounds into a double bond. Such selective partial hydrogenation can be performed only by using a suitable catalyst. One of the most widely used of such catalysts is a so-called Lindlar catalyst which is a palladium catalyst borne on a calcium carbonate carrier and partially poisoned or inactivated with lead. A practical improvement in the selectivity of hydrogenation with a Lindlar catalyst has been proposed, in which the catalyst is used in combination with the amine such as a quinoline, hexamethylenetetramine or a lower alkylamine (see, for example, Japanese Patent Publications Nos. 35-12762, 36-23317 and 38-64875). One of the problems in using amines is the contamination of the hydrogenated product with the amine. It is therefore very important that the amine should not have an unpleasant odour, especially when the product is to be used in perfumery where the smallest amount of any such contamination is wholly unsatisfactory.

Another problem inherent in the use of Lindlar catalysts lies in the separation and recovery of the catalyst after hydrogenation. Since the catalyst is borne on a calcium carbonate carrier, the catalyst is likely to be comminuted during the reaction and the separation of the finally powdered catalyst from the reaction mixture which is subsequently necessary is extremely difficult. Distillation of the reaction mixture containing finely powdered catalyst is sometimes undesirable, even if the amount of the powder is small, particularly when the product is to be used in perfumery.

According to the present invention, a method for the selective preparation of the *cis* isomer of an ethylenically unsaturated compound by the partial hydrogenation of a disubstituted acetylenically unsaturated compound comprises hydrogenating the acetylenic compound under pressure in the presence of water, a hydroxide or basic salt of an alkali metal or an alkaline earth metal, and a partially inactivated palladium catalyst borne on an alumina carrier.

The starting material used in the method of this invention is a disubstituted acetylenically unsaturated compound which can be represented by the formula $R^1C \equiv CR^2$, each of $R^1$ and $R^2$ being hydrocarbyl groups which are free of triple bonds and which may carry one or more substituents such as hydroxy, carboxyl, aldehyde or carbonyl oxygen groups or atoms. $R^1$ and $R^2$ may each be optionally substituted alkyl, cycloalkyl, alkenyl or aryl. $R^1$ and $R^2$ each usually contain from 1 to 20 carbon atoms.

Examples of suitable starting materials are 3-hexyn-1-ol, dehydrojasmone, 7-eicosyn-11-one, 1-phenyl-2-pentyn-1-ol, 2-propyn-1-ol, 2-butyne-1,4-diol and 6-heneicosyn-11-one.

In the catalyst used in this invention, the amount of palladium is from 3 to 15% by weight based on the total weight of the catalyst. If the amount of palladium is less than 3% by weight, the selectivity for, and the yield of, the *cis* isomer are lower and more of the saturated compound is formed. If the amount of palladium is higher than 15% by weight, palladium may be lost from the catalyst, and this is uneconomical.

The catalyst used in the invention is partially poisoned or inactivated with a poisoning element selected from zinc, lead and tin. The amount of the poisoning element is from 0.5 to 10% by weight, based on the total weight of the palladium and the alumina carrier. If the amount of the poisoning element is less than 0.5% by weight, the yield of the saturated compound increases. If the amount of the poisioning element is more than 10% by weight, the rate of the hydrogenation reaction is lower. The amount of the catalyst is preferably from 0.05 to 5% by weight, based on the weight of the disubstituted acetylenically unsaturated starting material. Amounts of catalyst below this range give low reaction velocity while amounts larger than 5% by weight give lower product selectivity. Two promoters are used in the present invention, i.e. water and an alkali or alkaline earth metal compound. The amount of water in the reaction mixture is preferably from 0.1 to 10% by weight, based on the weight of the disubstituted acetylenically unsaturated starting material. Amounts outside this range lead to lower yields of the desired product. The alkali or alkaline earth metal compound is a hydroxide or a basic salt of lithium, sodium, potassium, magnesium, calcium, strontium or barium. The basic salt may be, for example, a carbonate bicarbonate or acetate. The amount of this second promoter is preferably from 0.05 to 10% by weight, based on the weight of the acetylenic compound. Lower amounts lead to increased formation of the saturated compounds and decreased selectivity for the desired product while larger amounts can cause polymerisation of the starting material and/or the reaction product or the formation of undesirable by-products having an unpleasant odour.

The hydrogenation reaction is preferably carried out at from 20 to 200°C. Lower temperatures cause lower reaction velocity and lower selectivity for the desired product while higher temperature lead to increased amounts

of the saturated compound.

The pressure under which the reaction is conducted is not critical but is preferably from 0.1 to 10 MPa. Pressures below and above this range cause effects similar to those observed using temperatures below and above the preferred temperature range.

Of the following Examples, Examples 1 to 5 illustrative the invention while Examples A, B and C are comparative. The results of each Example are tabulated after the last Example.

## Example 1

50 g of 7-eicosyn-11-one, 0.1 g of a palladium catalyst containing 10% by weight of palladium on an alumina carrier and partially inactivated with 1.7% by weight of zinc, and 0.2 g of an aqueous solution containing 20% by weight of sodium hydroxide were introduced into a 200 ml autoclave. Hydrogen gas was then introduced to a pressure of 0.5 MPa and the reaction mixture was then agitated at 50°C, hydrogen then being absorbed at a rate of 0.1 1/min. The pressure was maintained at the starting value by continuously supplying hydrogen to the autoclave. When hydrogen absorption ceased, after 42 minutes, the agitation was stopped and the reaction mixture allowed to stand for 5 minutes, during which time the catalyst powder settled. After separating the reaction mixture and the catalyst by filtration, the filtrate was distilled to give a liquid product which was identified by gas chromatography to be *cis*-7-eicosen-11-one.

## Example 2

500 g of 3-hexyn-1-ol, 0.5 g of a palladium catalyst containing 10% by weight of palladium borne an alumina carrier and partially inactivated with 1.7% by weight of zinc, 0.5 g of anhydrous sodium carbonate and 1 g of water were introduced into a one litre capacity autoclave. Hydrogen gas was then introduced into the reaction vessel to a pressure of 0.5 MPa. This pressure was maintained while the reaction mixture was agitated at 50°C, whereupon hydrogen was absorbed at a rate of about 1 1/min. When hydrogen absorption ceased, after 125 minutes, the agitation was stopped and the reaction mixture allowed to stand for 5 minutes so that the catalyst powder settled. After removing the catalyst by filtration the reaction mixture was distilled to give a liquid product which was identified by gas chromatography to be mainly *cis*-3-hexen-1-ol.

## Example 3

The procedure of Example 1 was repeated, except that 0.1 g of barium hydroxide and 1.0 g of water were used instead of the sodium hydroxide solution. The absorption of hydrogen ceased after 42 minutes.

## Example A

The procedure of Example 2 was repeated, except that the anhydrous sodium carbonate was omitted.

## Example B

The procedure of Example 2 was repeated, except that the content of palladium in the catalyst was reduced to 1% by weight.

## Example C

The procedure of Example 2 was repeated, except that the palladium catalyst was calcium carbonate rather than alumina. It proved difficult to separate the catalyst from the reaction mixture after the reaction.

## Example 4

500 g of 3-hexyn-1-ol, 0.5 g of a catalyst containing 5% by weight of palladium borne on an alumina carrier and partially inactivated with 3% by weight of lead, 0.5 g of anhydrous sodium carbonate and 1.0 g of water were introduced into a one litre capacity autoclave. Hydrogenation was then conducted, substantially in the manner described in Example 2.

## Example 5

The procedure of Example 4 was repeated, except that the catalyst was partially inactivated with 3% by weight of tin rather than the same proportion of lead.

The results of gas chromatographic analysis of the reaction products of each of the preceding Examples are tabulated below: the yields of the desired products (*cis*-7-eicosen-11-one in Examples 1 and 3 and *cis*-3-hexen-1-ol in the other Examples) i.e. the *cis* isomer, the corresponding *trans* isomer and the corresponding saturated compound are given in weight percentages.

| Examples | cis Isomer | trans Isomer | Saturated Compound |
|---|---|---|---|
| 1 | 97.0 | 2.7 | 0.3 |
| 2 | 98.2 | 1.6 | 0.18 |
| 3 | 97.8 | 2.0 | 0.23 |
| A | 86.1 | 13.2 | 0.70 |
| B | 84.2 | 13.4 | 2.39 |
| C | 84.8 | 13.5 | 1.65 |
| 4 | 97.5 | 2.2 | 0.3 |
| 5 | 97.0 | 2.5 | 0.5 |

## Claims

1. A method for the selective preparation of the *cis* isomer of a disubstituted ethylenically unsaturated compound by the partial hydrogenation of a disubstituted compound by the partial hydrogenation of a disubstituted acetylenically unsaturated compound under pressure in the presence of a partially inactivated carrier-borne palladium catalyst, characterised in that the hydrogenation is conducted in the additional presence of water and a hydroxide or basic salt of an alkali metal or alkaline earth metal, and in that the catalyst comprises alumina carrying from 3 to 15% palladium partially inactivated with from 0.5 to 10% of a poisoning element selected from zinc, lead and tin, the percentages being by weight based on the total weight of the catalyst.

2. A method according to claim 1 in which the amount of water is from 0.1 to 10% by weight, based on the weight of the acetylenic compound.

3. A method according to claim 1 or claim 2 in which the basic salt is a carbonate, bicarbonate or acetate.

4. A method according to any preceding claim in which the amount of the hydroxide or basic salt is from 0.05 to 10% by weight, based on the weight of the acetylenic compound.

5. A method according to any preceding claim in which the amount of the catalyst is from 0.05 to 5% by weight, based on the weight of the acetylenic compound.

6. A method according to any preceding claim in which the hydrogenation is conducted at from 20 to 200°C under a hydrogen pressure of from 0.1 to 10 MPa.

## Revendications

1. Procédé pour la préparation sélective de l'isomère cis d'un composé disubstitué éthyl-èniquement insaturé, par hydrogénation partielle d'un composé disubstitué acétyléniquement insaturé, sous pression en présence d'un catalyseur au palladium partiellement inactive fixé sur un support, caractérisé en ce que l'hydrogénation est effectuée en outre en présence d'eau et d'un hydroxyde ou d'un métal alcalin ou alcalino-terreux, et en ce que le catalyseur comprend de l'alumine servant de support à entre 3 et 15% de palladium partiellement inactivé au moyen de 0,5 à 10% d'un élément poison choisi parmi le zinc, le plomb et l'étain, les pourcentages se rapportant au poids total du catalyseur.

2. Procédé selon la revendication 1, caractérisé en ce que la quantité d'eau est comprise entre 0,1 et 10 % en poids par rapport au poids du composé acétylènique.

3. Procédé selon la revendication 1 ou 2, caractérisé en ce que le sel basique est un carbonate, un bicarbonate ou un acétate.

4. Procédé selon l'une quelconque des revendications précédentes, caractérisé en ce que la quantité d'hydroxyde ou de sel basique est comprise entre 0,005 et 10% en poids par rapport au poids du composé acétylénique.

5. Procédé selon l'une quelconque des revendications précédentes, caractérisé en ce que la quantité de catalyseur est comprise entre 0,05 et 5% en poids, par rapport au poids du composé acétylénique.

6. Procédé selon l'une quelconque des revendications précédentes, caractérisé en ce que l'hydrogénation est effectuée entre 20 et 200°C sous une pression d'hydrogène comprise entre 0,1 et 10 MPa.

## Patentansprüche

1. Verfahren zur selektiven Herstellung des cis-Isomers einer disubstituierten ethylenisch ungesättigten Verbindung durch partielle Hydrierung einer disubstituierten acetylenisch ungesättigten Verbindung unter Druck in Gegenwart eines partiell inaktivierten trägergestützten Palladiumkatalysators, dadurch gekennzeichnet, daß die Hydrierung in der zusätzlichen Gegenwart von Wasser und einem Hydroxid oder basischen Salz eines Alkalimetalls oder Erdalkalimetalls durchgeführt wird und daß der katalysator Aluminiumoxid enthält, das von 3 bis 15% Palladium trägt, und der katalysator partiell inaktiviert ist mit 0,5 bis 10% eines vergiftenden Elements ausgewählt aus Zink, Blei und Zinn, wobei die Prozentangaben sich auf Gewicht bezogen auf das Gesamtgewicht des Katalysators beziechen.

2. Verfahren nach Anspruch 1, worin die Menge des Wassers von 0,1 bis 10 Gewichtsprozent bezogen auf das Gewicht der acetylenischen Verbindung beträgt.

3. Verfahren nach Anspruch 1 oder Anspruch 2, worin das basische Salz ein Carbonat, Bicarbonat oder Acetat ist.

4. Verfahren nach einem der vorangehenden Ansprüche, worin die Menge des Hydroxids oder basischen Salzes von 0,05 bis 10 Gewichtsprozent, bezogen auf das Gewicht der acetylenischen Verbindung, beträgt.

5. Verfahren nach einem der vorangehenden Ansprüche, worin die Menge des Katalysators von 0,05 bis 5 Gewichtsprozent, bezogen auf das Gewicht der acetylenischen Verbindung, beträgt.

6. Verfahren nach einem der vorangehenden Ansprüche, worin die Hydrierung bei 20 bis 200°C unter einem Wasserstoffdruck von 0,1 bis 10 MPa durchgeführt wird.